(19) Europäisches Patentamt
European Patent Office
Office européen des brevets

(11) Veröffentlichungsnummer : **0 093 343**
**B1**

(12) # EUROPÄISCHE PATENTSCHRIFT

(45) Veröffentlichungstag der Patentschrift : **05.03.86**

(51) Int. Cl.[4] : **C 07 D451/06**, A 61 K 31/46

(21) Anmeldenummer : **83103936.7**

(22) Anmeldetag : **22.04.83**

(54) **Verfahren zur Herstellung von Norscopin.**

(30) Priorität : **26.04.82 DE 3215490**

(43) Veröffentlichungstag der Anmeldung : **09.11.83 Patentblatt 83/45**

(45) Bekanntmachung des Hinweises auf die Patenterteilung : **05.03.86 Patentblatt 86/10**

(84) Benannte Vertragsstaaten : **AT BE CH DE FR GB IT LI LU NL SE**

(56) Entgegenhaltungen :
**DE-B- 1 670 257**
**CHEMICAL ABSTRACTS, Band 94, 1981, Seite 153, Nr. 59168e AND THE TENTH COLLECTIVE INDEX, Seite 35768 CS, Columbus, Ohio, USA, LONG-NAN CHAO: "A rapid method for the detection of some drugs mixed in Chinese herb preparations by thin-layer chromatography"**
**R.H.F. MANSKE et al.: "The alkaloids, chemistry and physiology, Band 1, 1950, Seiten 302-307, Academic Press Inc., Publishers, New York, USA**
**T.A. HENRY: "The plant alkaloids", 3. Auflage, 1939, Seiten 69-118, J. & A. Churchill Ltd., London, GB.**

(73) Patentinhaber : **BOEHRINGER INGELHEIM KG**

**D-6507 Ingelheim am Rhein (DE)**
**AT BE CH DE FR GB IT LI LU NL SE**
**Boehringer Ingelheim International G.m.b.H**

**D-6507 Ingelheim am Rhein (DE)**
**GB**

(72) Erfinder : **Banholzer, Rolf, Dr.**
**Johann-Calvin-Strasse 11**
**D-6507 Ingelheim/Rhein (DE)**

**EP 0 093 343 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von von Norscopin, Formel

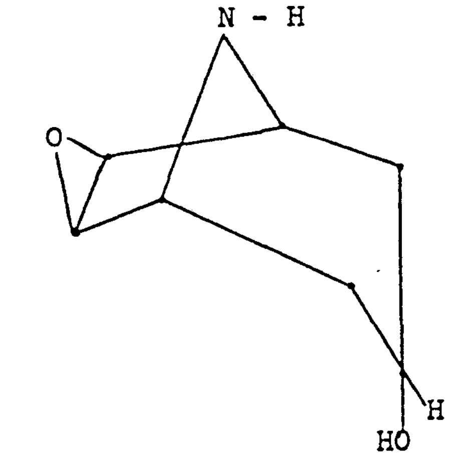

(I)

und seiner Säureadditionssalze.

Norscopin ist bereits in der Literatur erwähnt worden (Chem. Abstr. loth Collective Index, Seite 35 768 CS ; Columbus, Ohio, USA) und fällt auch unter den allgemeinen Anspruch der DE-A-1 670 257.

In « The Alkaloides », Seite 307 (R.H.F. Manske et al. ; Band 1 (1950), Academic Press Inc., New York, USA) und entsprechend in « The Plant Alkaloids », Seite 93 (T.A. Henry ; 3. Aufl. (1939) ; Churchill Ltd., London, GB) wird die teilweise oxidative Unwandlung von Scopolamin (neben der Bildung des N-Oxids) in Scopiniumbromid beschrieben, das dann mit Natriumamalgam in Pseudoscopin übergeführt wurde.

Die Übertragung dieses Verfahrens auf Norscopolamin erscheint jedoch nicht sinnvoll, da die Entstehung von Pseudonorscopin unerwünscht ist.

Erfindungsgemäß wird demgegenüber Norscopolamin reduktiv in Norscopin umgewandelt, wobei es nicht zu der störenden Isomerisierung zu Pseudonorscopin kommt.

Das Norscopin ist ein wertvolles Vorprodukt, beispielsweise zur Herstellung neuer 6,11-Dihydro-dibenzo-[b,e]-thiepin-11-N-alkyl-norscopinether der allgemeinen Formel

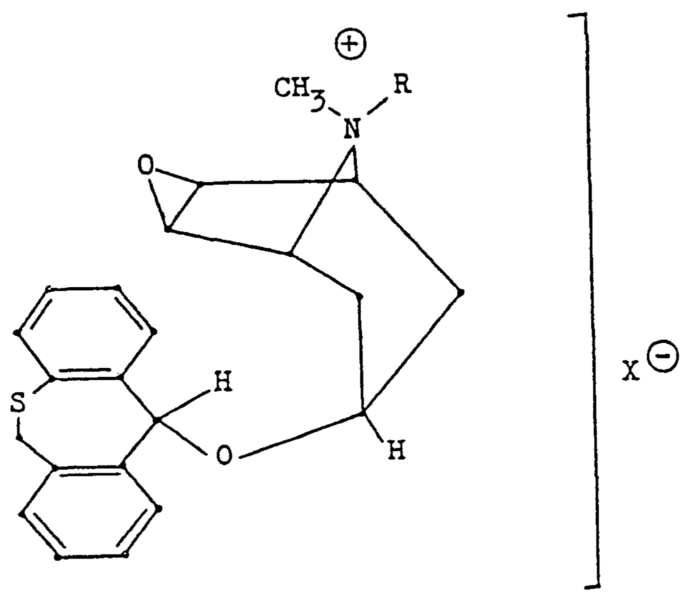

(II)

worin R einen geradkettigen oder verzweigten Alkylrest mit 1-10 Kohlenstoffatomen und X$^{(-)}$ ein pharmakologisch unbedenkliches Anion, wie etwa ein Halogenatom oder den Rest einer organischen Sulfonsäure, darstellt ; Diese Stoffe sind ihrerseits wertvolle Pharmazeutika mit einem gut ausgewogenen Verhältnis von anticholinerger und antihistaminischer wirkung. Sie eignen sich daher hervorragend für die Anwendung als Broncholytica. Diese Substanzen sind in der deutschen Patentanmeldung EP-AI-0 090 195 beschrieben.

Die Weiterverarbeitung des Norscopins zu den Norscopinethern der oben bezeichneten allgemeinen Formel II kann so erfolgen, daß man zunächst (z. B. durch übliche N-Alkylierung mit Alkylhalogeniden)

die N-Alkylnorscopine der allgemeinen Formel

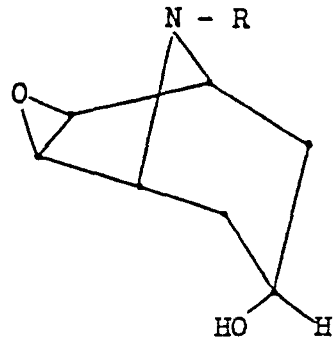

(III)

worin R die oben genannte Bedeutung hat, herstellt ; diese ihrerseits mit Verbindungen der allgemeinen Formel

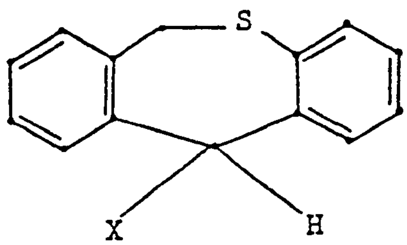

(IV)

worin X eine leicht abspaltbare Gruppe (z. B. ein Halogenatom) bedeutet, zu tertiären Verbindungen der allgemeinen Formel

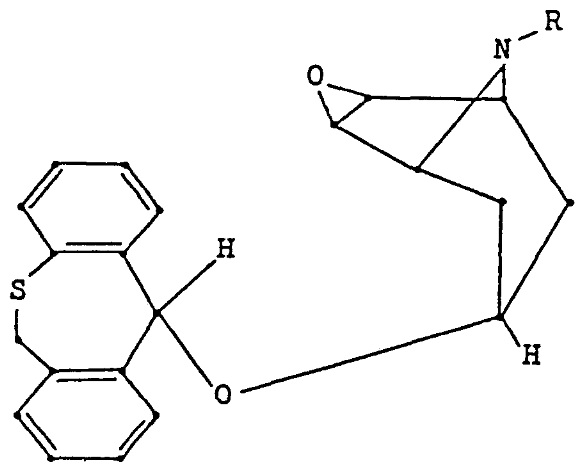

(V)

worin R die oben genannte Bedeutung hat, umsetzt und letztere wiederum mit üblichen Quaternierungsmitteln der allgemeinen Formel

$$R — X \qquad (VI),$$

worin R und X die oben genannte Bedeutung haben, in die Quartärsalze der allgemeinen Formel II überführt.

Bezüglich näherer Einzelheiten der Weiterverarbeitung zu Verbindungen der allgemeinen Formel II wird auf die bereits erwähnte deutsche Patentanmeldung EP-Al-0 090 195 verwiesen.

Die erfindungsgemäße Herstellung des Norscopins erfolgt so, daß man Norscopolamin bzw. seine Salze mit geeigneten komplexen Metallhydriden (vorzugsweise bei Rautemperatur oder niedrigeren Temperaturen) in einem Lösungsmittel behandelt. Besonders geeignet sind Metallborhydride und hier wiederum das Natriumborhydrid ($NaBH_4$). Auch Lithiumalanat ist grundsätzlich geeignet ; hierbei sollten jedoch zwecks Vermeidung von Nebenreaktionen Temperaturen von 0 °C und weniger angewandt werden.

Als Lösungsmittel für die vorbezeichnete Umsetzung kommt vor allem Ethanol in Betracht. Höhere Alkohole und andere organische Lösungsmittel (z. B. Ether) sind weniger günstig, da in ihnen die

vorzugsweise als Ausgangsprodukte verwendeten Norscopolaminsalze schwerlöslich sind. Auch Methanol ist nur wenig geeignet, da es beispielsweise Natriumborhydrid schon bei 0 °C sehr rasch zersetzt, so daß die Einsatzmenge dieses Reagens unnötig vergrößert wird. Auch in Wasser kann die Hydrogenolyse erfolgen, wenn z. B. der pH auf ca. 6-7 eingestellt wird. Die anschließende Isolierung des Norscopins aus Wasser ist jedoch mühsamer als die aus Ethanol.

Die Herstellung des als Ausgangsprodukt benutzten Norscopolamins kann den DE-AS 1 670 257 und 1 670 258 entnommen werden.

Das folgende Beispiel erläutert die Erfindung, ohne sie zu beschränken.

## Beispiel

Norscopin bzw. Norscopin-hydrochlorid

32,6 g (0,1 Mol) Norscopolamin-hydrochlorid werden in 350 ml Ethanol suspendiert und bei einer Temperatur von 20 °C unter ständigem Rühren in sechs Anteilen in Abständen von jeweils 20 Minuten mit 3,78 g (0,1 Mol) Natriumborhydrid versetzt. Man läßt 12 Stunden nachreagieren. Die Hydrogenolyse ist nach dieser Zeit beendet. Bei – 5 bis – 10 °C wird nun unter Stickstoffatmosphäre Chlorwasserstoffgas bis zur sauren Reaktion eingeleitet, die Lösung mit 1,5 Liter Ether frei von Chlorwasserstoff gewaschen. Die nach dem Trocknen fein verriebenen Kristalle suspendiert man in 2 Liter Methylenchlorid, erhitzt zum Sieden und leitet in das siedende Methylenchlorid zur Bildung der Base Ammoniak bis zur völligen Umsetzung ein. Nach dem Abtrennen der organischen Salze wird das Methylenchlorid unter vermindertem Druck bei 30 °C abdestilliert.

Das Norscopin kristallisiert in farblosen Kristallen. Schmelzpunkt 198-202 °C (Zers.)

Ausbeute : 12,0 g (85,7 % d. Th.)

Das Hydrochlorid kann nach üblichen Methoden hergestellt werden.

Farblose Kristalle (Ethanol), Schmelzpunkt 292-293 °C (Zers.), Umwandlungspunkt ~ 205 °C).

Durch Elementaranalyse und Spektren wird das Vorliegen dieser Verbindung bestätigt.

## Patentansprüche

1. Verfahren zur Herstellung von Norscopin und seinen Salzen, dadurch gekennzeichnet, daß man Norscopolamin bzw. seine Salze in einem geeigneten Lösungsmittel mit einem komplexen Hydrid hydrogenolytisch spaltet.
2. Verfahren nach Anspruch 1, dadurch gekennzeichnet, daß man als komplexes Hydrid ein Metallborhydrid einsetzt.
3. Verfahren nach Anspruch 2, dadurch gekennzeichnet, daß man mit Natriumborhydrid bei Raumtemperatur in Ethanol arbeitet.

## Claims

1. Process for the preparation of norscopine and the salts thereof, characterised in that norscopolamine or a salt thereof is hydrogenolytically cleaved with a complex hydride in a suitable solvent.
2. Process as claimed in claim 1, characterised in that a metal borohydride is used as the complex hydride.
3. Process as claimed in claim 2, characterised in that the work is done with sodium borohydride at ambient temperature in ethanol.

## Revendications

1. Procédé pour la préparation de la norscopine et de ses sels, caractérisé en ce que l'on clive hydrogénolytiquement la norscopolamine ou ses sels dans un solvant approprié au moyen d'un hydrure complexe.
2. Procédé selon la revendication 1, caractérisé en ce que l'on utilise en tant qu'hydrure complexe un borohydrure métallique.
3. Procédé selon la revendication 2, caractérisé en ce que l'on travaille avec du borohydrure de sodium à la température ambiante dans l'éthanol.